# EUROPEAN PATENT SPECIFICATION

(11) **EP 0 605 753 B1**
(45) Date of publication and mention of the grant of the patent: **21.01.2004**
(21) Application number: 93105258.3
(22) Date of filing: 30.03.1993
(51) Int. Cl.: A61K 47/48, A61K 31/337, A61P 35/00

(54) **Cyclodextrin inclusion complex of taxol, and method for its production and its use**
Einschlusskomplex von Taxol, Verfahren zu seiner Herstellung und Verwendung
Complexe d'inclusion du taxol, procédé pour sa préparation et utilisation

(30) Priority: 27.11.1992 JP 33949592
(43) Date of publication of application: 13.07.1994
(73) Proprietor: Ensuiko Sugar Refining Company, Limited, Kanagawa-ken,230 (JP)
(72) Inventor: Mikuni, Katsuhiko, c/o Ensuiko Sugar Refining Co.,, Yokohama-shi, Kanagawa-ken (JP); Kuwahara, Nobuhiro, c/o Ensuiko Sugar Refining, Yokohama-shi, Kanagawa-ken (JP); Hamada, Hiroki, c/o Okayamarika-daigaku,, Okayama-ken (JP); Saito, Kyoko, c/o Okayamarika-daigaku,, Okayama-ken (JP)
(74) Representative: VOSSIUS & PARTNER

(56) References cited:
- EP-A- 0 519 428
- EP-A- 0 522 936
- EP-A- 0 522 937
- WO-A-91/04026
- WO-A-92/09589
- DE-A- 4 028 004
- FR-A- 2 484 252
- ACTA PHARMACEUTICA, vol.36, no.1, March 1990, STUTTGART, DE pages 1 - 6 D. DUCHENE ET AL 'The Current State of beta-Cyclodextrin in Pharmaceutics'
- KOIZUMI ET AL: 'Inclusion complexes of poorly water-soluble drugs with glucosyl-cyclodextrins' CHEM. PHARM. BULL. vol. 35, no. 8, pages 3413 - 3418
- OKADA ET AL: 'Some properties and the inclusion behavior of branched Cyclodextrins' CHEM. PHARM. BULL. vol. 36, no. 6, pages 2176 - 2185

## Description

The present invention relates to a method for improving the solubility of of paclitaxel in water, and more particularly, to a method for improving its water solubility by adding cyclodextrin or branched cyclodextrin (both sometimes referred to hereafter as "CD" at a molar ratio of 1-20 times with respect to paclitaxel to obtain a CD inclusion complex of paclitaxel, thus improving the solubility of paclitaxel in water and solubilizing paclitaxel.

Paclitaxel, also known under the registered trade mark "Taxol®" and herienafter referred to as taxol, is a substance which is extracted from the bark of a

species of the North American yew tree (Taxus brevifolia), and it inhibits the division of cancer cells. Particularly, it is known to be effective on ovarian cancer patients, and is attracting attention as a novel and powerful anti-cancer agent. However, taxol does not dissolve in water, and thus is not absorbed when administered to patients.

Taxol was only recently discovered, and no reports exist as yet regarding its solubility.

Thus, the object of the present invention is to overcome the above mentioned disadvantage of taxol.

This object has been achieved by the surprising finding that taxol can be solubilized by adding CD thereto at a molar ratio of 1-20 times with respect to taxol. Therefore, the present invention provides a cyclodextrin or branched cyclodextrin inclusion complex of taxol, and further provides a method for the production of a cyclodextrin or branched cyclodextrin inclusion complex of taxol characterized by adding cyclodextrin or branched cyclodextrin to taxol at a molar ratio of 1-20 times with respect to taxol, a method for the improvement of the solubility of taxol in water by adding cyclodextrin or branched cyclodextrin thereto at a molar ratio of 1-20 times with respect to taxol, a pharmaceutical composition containing said inclusion complex of taxol, as well as the use of the inclusion complex of taxol for the preparation of an anti-cancer agent.
Fig. 1 shows an HPLC chromatogram of taxol.
Fig. 2 shows an absorption spectrum of taxol.
Fig. 3 shows an HPLC chromatogram of taxol-CD in a case where ethanol was used as the solvent according to Example 2.
Fig. 4 shows an absorption spectrum of taxol-CD in a case where ethanol was used as the solvent according to Example 2.
Fig. 5 shows a three-dimensional HPLC chromatogram of taxol-maltosyl-B-CD in a case where ethanol was used as the solvent according to Example 2.
Fig. 6 shows an HPLC chromatogram of taxol-γ-CD in a case where ethyl acetate was used as the solvent and manual reciprocal shaking was effected according to Example 2.
Fig. 7 shows an absorption spectrum of taxol-γ-CD in a case where ethyl acetate was used as the solvent and manual reciprocal shaking was effected according to Example 2.
Fig. 8 shows an HPLC chromatogram of taxol-γ-CD in a case where ethyl acetate was used as the solvent and stirring was effected for one hour according to Example 3.
Fig. 9 shows an absorption spectrum of taxol-γ-CD in a case where ethyl acetate was used as the solvent and stirring was effected for one hour according to Example 3.
In order to obtain a CD inclusion complex of taxol, taxol is first dissolved in an organic solvent. Here, the organic solvent may be ethyl acetate, methanol, ethanol, acetone, etc., but ethanol is particularly preferable. Here, the amount of the organic solvent is not particularly limited so far as it dissolves the taxol.

On the other hand, an aqueous solution of CD is prepared in an amount of 1-20 times the molarity of the taxol, and is added to the taxol solution. In this point, stirring is qenerally preferred for complete mixing of the solution, and more preferably, a stirrer or the like is used for vigorous stirring. Also, there are no special conditions regarding the reaction temperature, and the reaction may proceed adequately at room temperature. The reaction time may be from a few minutes to a few hours, and is normally from about 2 minutes to an hour.

Taxol is clathrated (or included) in the CD by this reaction. The CD used according to the present invention may be, for example, α-CD, β-CD, γ-CD, glucosyl-α-CD, glucosyl-β-CD, glucosyl-γ-CD, maltosyl-α-CD, maltosyl-β-CD, maltosyl-γ-CD, maltotriosyl-α-CD, maltotriosyl-β-CD, maltotriosyl-γ-CD, etc., and these may be used alone or in appropriate admixture.

If the amount of CD added is less than the lower limit of 1:1 with respect to taxol, the solubility of taxol is not adequately increased, and if it is greater than 1:20, the excessive amount of CD does not contribute to inclusion complex formation.

In addition, if the solution is dried, a stable CD inclusion complex of taxol may be obtained, which is highly soluble in water.

When a CD inclusion complex of taxol obtained in this manner is administered to a patient intravenously, orally or via another route, a minute but highly efficient dosage of taxol may be provided to the patient. Thus, the physiological activity of taxol may be efficiently induced.

A CD inclusion complex of taxol obtained according to the present invention may be used in a variety of different forms. For example, it may be prepared as an injection or used as a powder without further processing, or the powder may be granulated, tableted or filled into capsules, etc., in a form as to best accomodate the patient. Of course, it may also be used in the form of the original solution. Further, the same effects may be expected even if taxol is clathrated using a chemically modified CD such as hydroxypropyl CD.

### Example 1

In this example, the influence of each type of CD on the solubilization of taxol was investigated.

First, 1 mg of taxol was dissolved in 2.0 ml of ethyl acetate. Meanwhile, each type of CD was dissolved in 2.0 ml of water to a molar ratio (taxol:CD) of 1:1, 1:5, 1:10 and 1:20, respectively. The CDs used were α-, γ-, maltosyl-α-, maltosyl-β- and maltosyl-γ-CD.

The taxol solution and the CD solutions were placed in test tubes each equipped with a ground-in stopper, and were then mixed by manual reciprocal shaking repeated 30 times. Next, the ethyl acetate layer was collected, and the absorbance was measured at 255 nm to calculate the amount of taxol which moved to the aqueous layer. The solubility of taxol was calculated by subtracting the solubility thereof when CD was not dissolved in the aqueous layer (blank value), and the results are shown in Table 1. That is, since ethyl acetate used here moves to the aqueous phase and thus a minute amount of taxol dissolves therein even in a blank case where no CD is used, a solubility of 0.589 mg per 2 ml of water is obtained, and therefore this value has been subtracted from the solubilities listed in Table 1. The numbers in the table, then, represent the solubility of taxol increased by the addition of CD.

**Table 1**

| Increased solubility of taxol in water using various CDs | | | | |
|---|---|---|---|---|
| Molar ratio (taxol:CD) | 1:1 | 1:5 | 1:10 | 1:20 |
| α-CD | 0.067 | 0.036 | 0.213 | 0.186 |
| γ-CD | 0.045 | 0.050 | 0.315 | 0.319 |
| Maltosyl-α-CD | 0.048 | 0.102 | 0.106 | 0.208 |
| Maltosyl-β-CD | 0.036 | 0.017 | 0.120 | 0.132 |
| Maltosyl-γ-CD | 0.055 | 0.044 | 0.183 | 0.118 |
| (mg per 2ml of water) | | | | |

The above results show that the solubility of taxol is improved by addition of CD at a molar ratio thereto of 1:1 or greater. Also, with the exception of maltosyl-α-CD, an adequate effect was obtained with addition of CD at a molar ratio of 1:10 or greater.

Further, of the various types of CD, γ-CD in particular produced significant improvement in the solubility of taxol, identifying it as the most appropriate substance for solubilization of taxol.

### Example 2

In this example, the solubility of CD inclusion complex of taxol was determined in a more precise manner than in Example 1, and the influence of the organic solvent used to dissolve taxol was also investigated.

One milligram of taxol was dissolved in 2.0 ml of ethanol, and each type of CD was dissolved in 2.0 ml of water to a molar ratio (taxol:CD) of 1:1, 1:5, 1:10 and 1:20, respectively. The CDs used were α-, γ-, maltosyl-α-, maltosyl-β- and maltosyl-γ-CD.

The taxol solution and the CD solution were placed in a test tube equipped with a ground-in stopper, and mixed by manual reciprocal shaking repeated 30 times, in the same manner as in Example 1. The entire solution was frozen immediately thereafter, and then subjected to lyophilization, which completely removed the water and the organic solvent.

Next, 2.0 ml of water were added to each of the lyophilized products obtained in this manner, the mixture was stirred and centrifuged, and then the solubility of the taxol in the supernatant was measured by high performance liquid chromatography (HPLC). The results thereof are shown in Table 2, along with the results of a case which was effected in the same manner, but using 2.0 ml of ethyl acetate as the organic solvent in place of 2.0 ml of ethanol.
The conditions for HPLC were as follows.
- Column:: Crestpack (Jasco)
- Solvent:: Methanol:water = 60:40
- Flow rate:: 1.0 ml/min
- Column temperature:: 40°C
- Detector:: Ultraviolet detector

**Table 2**

| Influence of solvent on solubility of taxol in water | | |
|---|---|---|
| | Ethyl acetate | Ethanol |
| α-CD | 0.4 | 14.5 |
| γ-CD | 10.8 | 117.9 |
| Maltosyl-α-CD | 3.1 | 11.1 |
| Maltosyl-β-CD | 4.4 | 42.8 |
| Maltosyl-γ-CD | 8.1 | 38.0 |
| (µg per mg of water) | | |

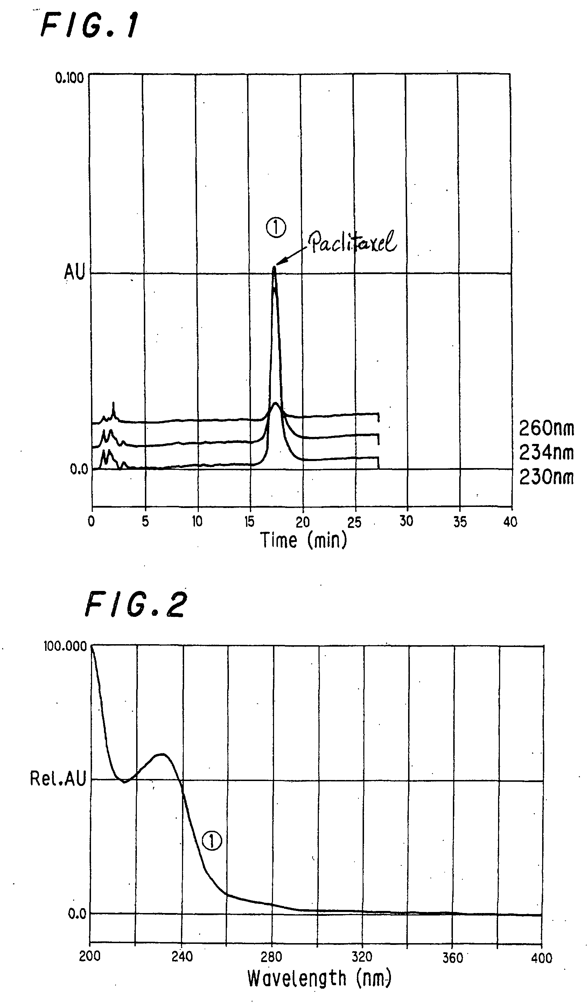

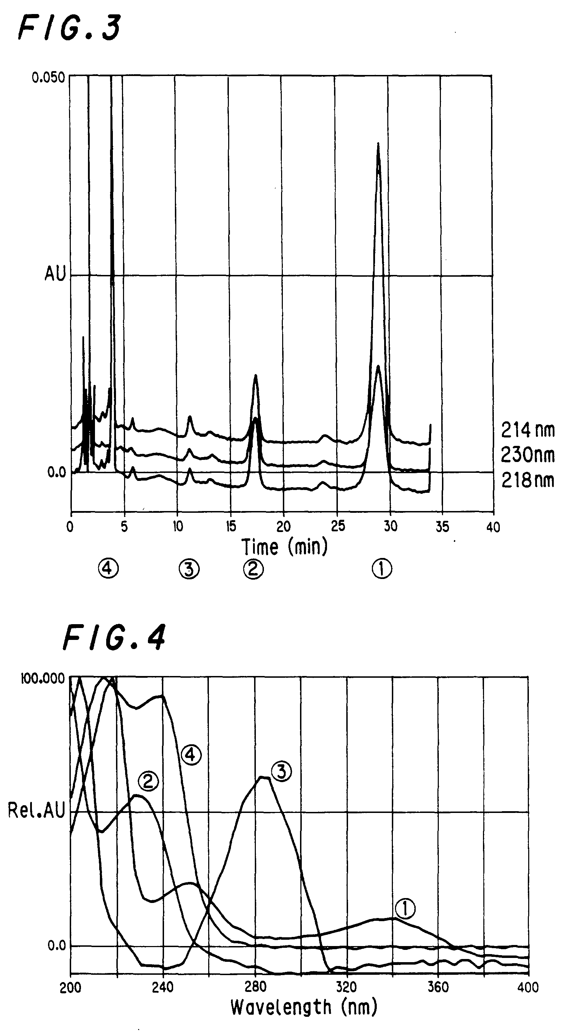

For the taxol assay, the taxol itself untreated with CD was dissolved in ethyl acetate, and taxol solutions of various concentrations were prepared and their HPLC chromatograms were prepared. One example thereof is shown in Fig. 1, and the absorption spectrum is shown in Fig. 2. On the other hand, the HPLC chromatogram of a case where ethanol and γ-CD were used is shown in Fig. 3, and the absorption spectrums at each peak measured at the same times as indicated in Fig. 3 are shown in Fig. 4. The peak picking information for Figs. 1 and 3 are given below.

| | Time (min) | AUFS | Peak wavelength |
|---|---|---|---|
| Fig. 1 (1) | 17.27 | 0.078 | 230 |
| Fig. 3 (1) | 28.95 | 0.038 | 218 |
| Fig. 3 (2) | 17.27 | 0.015 | 230 |
| Fig. 3 (3) | 11.26 | 0.005 | 284 |
| Fig. 3 (4) | 3.86 | 0.036 | 214 |

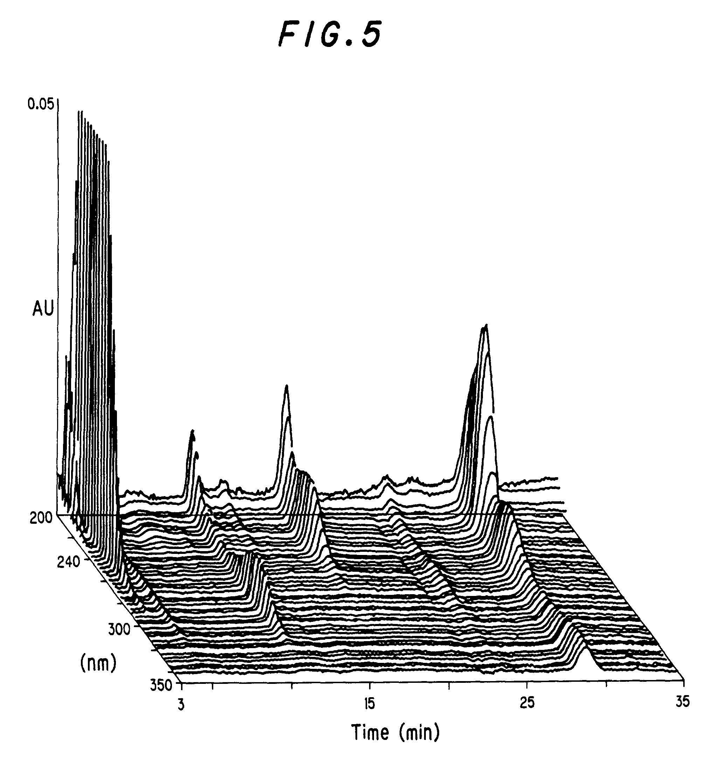

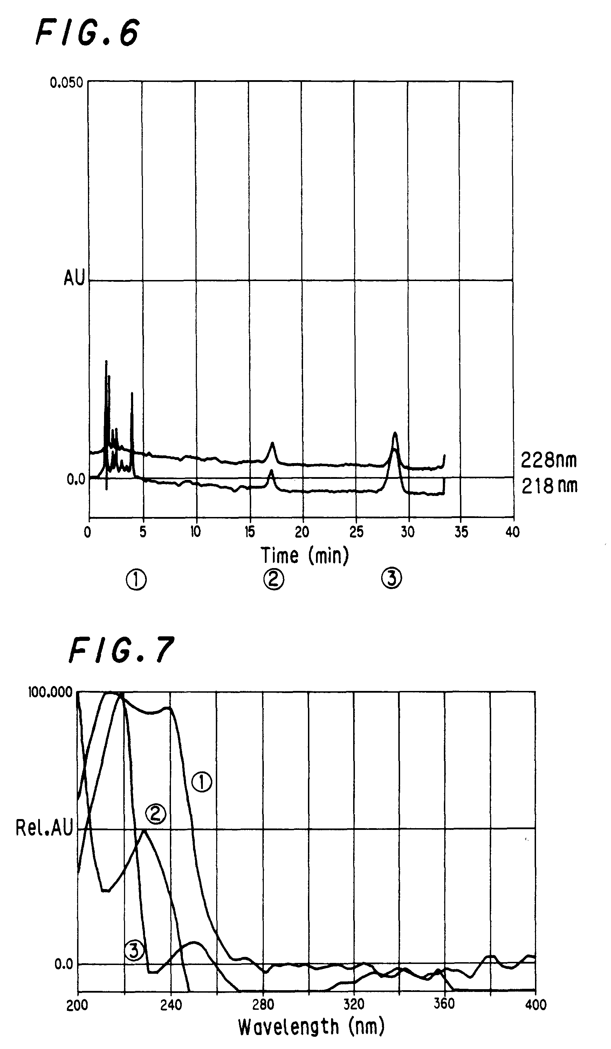

In addition, a three-dimensional HPLC chromatogram of a case where ethanol and maltosyl-β-CD were used is shown in Fig. 5, and an HPLC chromatogram of a case where ethyl acetate and γ-CD were used is shown in Fig. 6. The absorption spectrum measured at each peak at the times indicated in Fig. 6 are shown in Fig. 7. The peak picking information for Fig. 6 is given below.

| | Time (min) | AUFS | Peak wavelength |
|---|---|---|---|
| Fig. 6 (1) | 3.86 | 0.008 | 214, 238 |
| Fig. 6 (2) | 17.02 | 0.004 | 228 |
| Fig. 6 (3) | 28.78 | 0.006 | 218 |

As is clear from the Figures, the taxol to which CD was added had a different retention time and a different absorption spectrum in HPLC, compared to taxol by itself. This fact indicates inclusion of the functional groups of taxol by CD. It is thought that CD forms inclusion complex with the hydrophobic group of taxol, thus improving the solubility thereof. There was a greater improvement in the solubility when ethanol was used as the solvent for taxol, than when ethyl acetate was used. This is thought to be due to the changing of the conformation of taxol by the solvent. Even when ethanol was used as the solvent instead of ethyl acetate, γ-CD significantly improved the solubility of taxol, and was the most excellent solubilizer thereof.

### Example 3

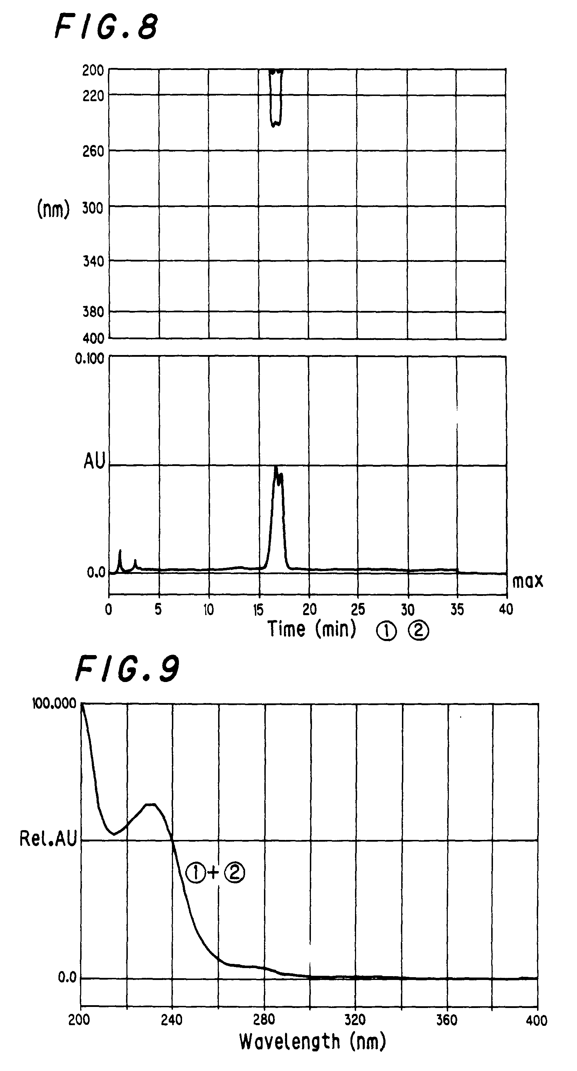

Twenty milligrams of taxol were dissolved in 40 ml of ethyl acetate, 200 mg of γ-CD were dissolved in 40 ml of water, and the two solutions were mixed and stirred for 1 hour with a stirrer. After allowing the mixture to stand, the aqueous layer was recovered and lyophilized. To the obtained dried product were added 40 ml of water, and the mixture was stirred for dissolution and subjected to centrifugation, after which the amount of taxol contained in the supernatant was measured using HPLC. The conditions for HPLC were the same as those in Example 2. The results are shown in Fig. 8, and the absorption spectrums measured at the respective peaks at the same times as indicated in Fig. 8 are shown in Fig. 9. The peak picking information for Fig. 8 is given below.

| | Time (min) | AUFS |
|---|---|---|
| Fig. 8 (1) | 16.45 | 0.041 |
| Fig. 8 (2) | 17.19 | 0.046 |

The results indicated an amount of taxol of 81.5 µg per 1 mg of water. Comparing this to the result of 10.8 µg in Example 2 where manual reciprocal shaking was repeated 30 times, there was clearly a drastic increase in solubility. The reason for this is thought to be that the stirring time (reaction time) was 1 hour, or longer than in Example 2.

Further, when the HPLC chromatograms are compared, elution was effected at retention times 3.86, 17.02 and 28.78 minutes (Fig. 6) in the case where manual reciprocal shaking was effected, whereas elution was effected at 16.45 and 17.19 minutes (Fig. 8) in the case where stirring was effected for one hour. Further, the absorption spectrums converged at around 230 nm (Fig. 9). This was though to be due to the fact that a clathration equilibrium was reached with a certain proportion of CD forming inclusion complexes of the functional groups of taxol.

Thus, with a reaction time of one hour, a CD inclusion equilibrium may be reached, the solubility of taxol may be improved, and the solubilization of taxol may be generally increased.

As mentioned above, according to the present invention, the solubility of taxol in water may be improved, and taxol may be solubilized, by adding CD thereto at a molar ratio of 1-20 times with respect to taxol. Therefore, if a CD inclusion complex of taxol according to the present invention is used, taxol administered to cancer patients may be easily absorbed, and the physiological effects of taxol may be more effectively induced.

## Claims

1. A cyclodextrin or branched cyclodextrin inclusion complex of paclitaxel, wherein the molar ratio of cyclodextrin to paclitaxel is in a range of from 1:1 to 20:1.

2. A method for the production of the inclusion complex of paclitaxel as defined in claim 1, **characterized by** adding cyclodextrin or branched cyclodextrin to paclitaxel at a molar ratio of 1 to 20 times with respect to paclitaxel.

3. A method for the improvement of the solubility of paclitaxel in water by adding a cyclodextrin or branched cyclodextrin to paclitaxel at a molar ratio of 1 to 20 times with respect to paclitaxel.

4. A pharmaceutical composition containing the inclusion complex of paclitaxel as defined in claim 1 and optionally a pharmaceutical carrier and/or diluent.

5. Use of the inclusion complex of paclitaxel as defined in claim 1 for the preparation of an anti-cancer agent.

## Patentansprüche

1. Einschlußkomplex von Paclitaxel mit Cyclodextrin oder verzweigtem Cyclodextrin, wobei das Molverhältnis von Cyclodextrin zu Paclitaxel im Bereich von 1: 1 bis 20:1 liegt.

2. Verfahren zur Herstellung des Einschlußkomplexes von Paclitaxel nach Anspruch 1, **dadurch gekennzeichnet, dass** Cyclodextrin oder verzweigtes Cyclodextrin zu Paclitaxel in einem 1- bis 20fachen Molverhältnis, bezogen auf Paclitaxel, gegeben wird.

3. Verfahren zur Verbesserung der Löslichkeit von Paclitaxel in Wasser durch Zugabe eines Cyclodextrins oder verzweigten Cyclodextrins zu Paclitaxel in einem 1- bis 20fachen Molverhältnis, bezogen auf Paclitaxel.

4. Arzneimittel, das den Einschlußkomplex von Paclitaxel nach Anspruch 1 und gegebenenfalls einen pharmazeutischen Träger und/oder ein Verdünnungsmittel enthält.

5. Verwendung des Einschlußkomplexes von Paclitaxel nach Anspruch 1 zur Herstellung eines Mittels gegen Krebs.

## Revendications

1. Complexe d'inclusion cyclodextrine ou cyclodextrine ramifiée du paclitaxel, dans lequel le rapport molaire de la cyclodextrine au paclitaxel est compris dans une gamme de 1:1 à 20:1.

2. Procédé de production du complexe d'inclusion du paclitaxel tel que défini dans la revendication 1, **caractérisé par** une addition de cyclodextrine ou cyclodextrine ramifiée au paclitaxel avec un rapport molaire de 1 à 20 fois par rapport au paclitaxel.

3. Procédé d'amélioration de la solubilité du paclitaxel dans l'eau par addition d'une cyclodextrine ou cyclodextrine ramifiée au paclitaxel avec un rapport molaire de 1 à 20 fois par rapport au paclitaxel.

4. Composition pharmaceutique contenant le complexe d'inclusion du paclitaxel tel que défini dans la revendication 1 et éventuellement un support pharmaceutique et/ou un diluant.

5. Utilisation du complexe d'inclusion du paclitaxel tel que défini dans la revendication 1 pour la préparation d'un agent anti-cancéreux.
